# EUROPEAN PATENT SPECIFICATION

(11) **EP 1 669 029 B1**
(45) Date of publication and mention of the grant of the patent: **05.08.2009**
(21) Application number: 05257550.3
(22) Date of filing: 08.12.2005
(51) Int. Cl.: A61B 8/00, G01S 7/52

(54) **Ultrasonic imaging apparatus with on-screen tool for measurement of feature geometry**
Ultraschall-Diagnostikgerät mit Hilfsmittel für die geometrische Vermessung eines auf einem Bildschirm dargestellten Merkmals
Appareil de diagnostic à ultrasons avec instrument de mesure de caracteristiques géometriques à l'aide d'élements affichés à l'écran

(30) Priority: 10.12.2004 JP 2004357517
(43) Date of publication of application: 14.06.2006
(73) Proprietor: GE Medical Systems Global Technology Company, LLC, Waukesha, Wisconsin 53188-1696 (US)
(72) Inventor: Mayumi, Ito, Hino-shi Tokyo 191-8503 (JP); Abe, Yayoi, Hino-shi Tokyo 191-8503 (JP); Fujiwara, Chiori, Hino-shi Tokyo 191-8503 (JP); Yawata, Tsutomu, Hino-shi Tokyo 191-8503 (JP)
(74) Representative: Bedford, Grant Richard

(56) References cited:
- US-A- 5 690 111
- US-A1- 2003 105 400
- US-B1- 6 464 640
- US-B1- 6 674 879

## Description

The present invention as defined by claim 1 is directed to an ultrasonic imaging apparatus, which displays ultrasonic image of a subject, and displays the measurement pattern in said displayed ultrasonic image based on the measurement pattern information with respect to the location and form of the measurement pattern to be input in order to perform the image measurement of said ultrasonic image.

Recently, in the ultrasonic imaging apparatus, the ultrasonic image, as typically represented by the 'B' mode image, has finer grains. In addition, not only the morphological examination in the real time basis but also the measurement of the organ anatomy as well as the examination based on the quantitative information derived therefrom can be performed.

In the quantitative examination, the measurement pattern including such as the trace are loaded in the ultrasonic image, and the pattern will be relocated to the target position by using a pointing device such as a track-ball for the image measurement (see for example JP-A-06-178773 pp 1 & Fig. 1).

Furthermore, the quantitative examination by means of the measurement pattern can be performed not only within a single ultrasonic image but also for the data comparison among a plurality of ultrasonic images, each of which may have a different imaging location or the acquisition time. For instance, in the cardiac dynamic examination using an ultrasound imaging apparatus a plurality of ultrasonic images for the atriums and ventricles in the diastolic and systolic times are displayed. For each of these ultrasonic images a measurement pattern will be set to gather and compare the numerical data.

In accordance with the prior art as have been described above, however, the data comparison using the measurement pattern can be done for the numerical data, and is neither instinctive nor visual. On the other hand, when performing a visual comparison of anatomical morphologies of measurement pattern set to each image by appositing a plurality of ultrasonic images of an imaging location sequencing the dynamism, the morphological comparison between measurement patterns may become too ambiguous to achieve a reliable morphological comparison.

In particular, when measuring the cardiac ultrasonic images using the measurement pattern, the parameters computed from the measurement pattern in a plurality of ultrasonic images can be multitudinous. When comparing among those parameters, it is important for an operator to precisely recognize the comparative morphology of the measurement pattern itself in order to valuably perform an appropriate examination with respect to the values of various parameters.

It is imperative therefore how to achieve an ultrasonic imaging apparatus, which allows the morphological comparison of the measurement pattern set within a plurality of ultrasonic images to be done in a reliable and easier manner.

In US-A-5 690 111 an ultrasound diagnostic apparatus which produces an overlaid image containing an ROI marker, comprises a frame combining circuit for combining ultrasonic images and the overlaid image into one frame, a display monitor, a CPU for changing the position of the ROI marker in the overlaid image, and a measuring circuit for obtaining measured values in the position of the ROI marker.

Document US5690111 discloses an ultrasonic imaging apparatus which displays an ultrasonic image of a subject, and also displays a measurement pattern , or ROI (region of interest) marker, in the displayed ultrasonic image, based on measurement pattern information with respect to a location of the measurement pattern to be input, to perform the image measurement the ultrasonic image, the ultrasonic imaging apparatus comprising: a selector device for specifying the location in a first ultrasonic image; image measurement means for calculating morphological information of the measurement pattern; a measurement pattern storage device for storing the morphological information of the measurement pattern; a measurement pattern reproduction device for reproducing the stored measurement pattern of the morphological information at a location in the first ultrasonic image specified using said selector device; a measurement pattern information buffer memory for storing measurement pattern information on the position of the measurement pattern; the measurement pattern comprising an ROI marker; the morphological information of the measurement pattern including an area on the basis of pixel data in the region of interest; measurement pattern moving means for changing the measurement pattern positional information in the measurement pattern information buffer memory; and the measurement pattern reproduction device being for reproducing the stored measurement pattern of the morphological information in a second ultrasonic image different from the first ultrasonic image ("overlaid image").

Document US6464640 discloses an ultrasound device with multiple region/s of interest selected by the operator on the same or different images acquired at different times and in which the ROI is "selected" within each image; and in which the microprocessor uses the ROI to create further so-called microprocessor regions of interest: but does not disclose feature that the measurement pattern information buffer stores the form of the measurement pattern.

In US-B1-6 674 879 a digital image processing system for enhancing the image quality and diagnostic capabilities of conventional medical diagnostic ultrasound imaging systems provides edge detection and automatic diagnostic features. In US2003/105400 an ultrasonic diagnostic apparatus utilizes two display devices with at least two display modes.

Therefore, an object of the present invention as defined by claim 1, is to provide an ultrasound imaging apparatus, which allows the morphological comparison of the measurement pattern set within a plurality of ultrasonic images to be done in a reliable and easier manner.

Further aspects of the invention are defined in the dependent claims.

Also disclosed in connection with the present invention, as defined by claim 1, the selector means specifies the position within an ultrasonic image, and if any measurement pattern is present at the specified location, the measurement pattern storage means will store the morphological info of the measurement pattern, and the measurement pattern reproduction means will reproduce thus stored morphological information of the measurement pattern at the position to be specified later by means of the selector means into an ultrasonic image, so that when performing the image measurement of imaged site where the morphology changes along with time course to compare the morphologies, not only numerical data but also the measurement pattern that indicates the form of imaged site at various temporal phase can be superposed to compare between the morphologies visually and reliably, and ultimately leading to a proper examination of numerical data by the operator.

Further objects and advantages of the present invention will be apparent from the following description of the preferred embodiments of the invention as illustrated in the accompanying drawings, in which:
Fig. 1 is a schematic block diagram illustrative of the overview of ultrasonic imaging apparatus.
Fig. 2 is a functional block diagram indicative of the functional structure of an image display function section and controller unit in accordance with the preferred embodiment.
Fig. 3 is a schematic diagram illustrative of an example of measurement pattern.
Fig. 4 is an external appearance view illustrative of a specific example of selector means in accordance with the preferred embodiment.
Fig. 5 is a flow chart indicative of the operation of controller unit in accordance with the preferred embodiment.
Fig. 6 is a schematic diagram illustrative of the storage and reproduction of a measurement pattern in the B mode image.
Fig. 7 is a schematic diagram illustrative of the sequential storage and sequential reproduction of a measurement pattern in the B mode image.
Fig. 8 is a schematic diagram illustrative of the batch storage and batch reproduction of a measurement pattern in the B mode image.

The best mode for carrying out the ultrasonic imaging apparatus in accordance with the present invention will be described in greater details herein below with reference to the accompanying drawings. It should be noted here that the illustrated embodiment are intended not to limit the present invention.

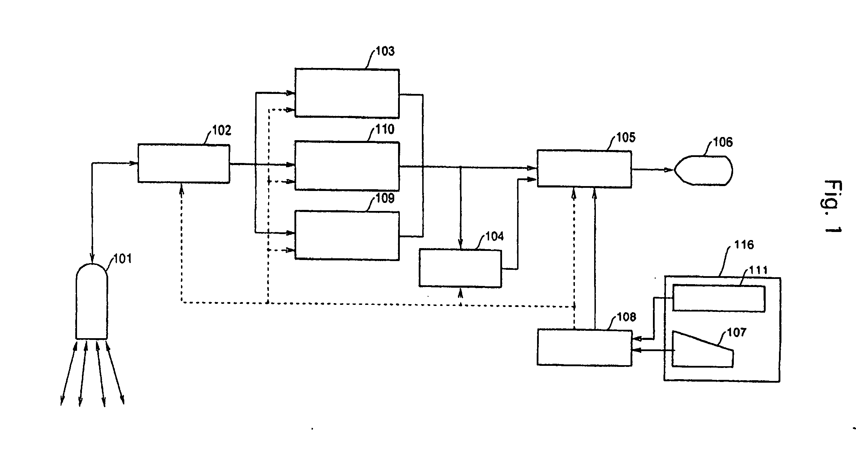

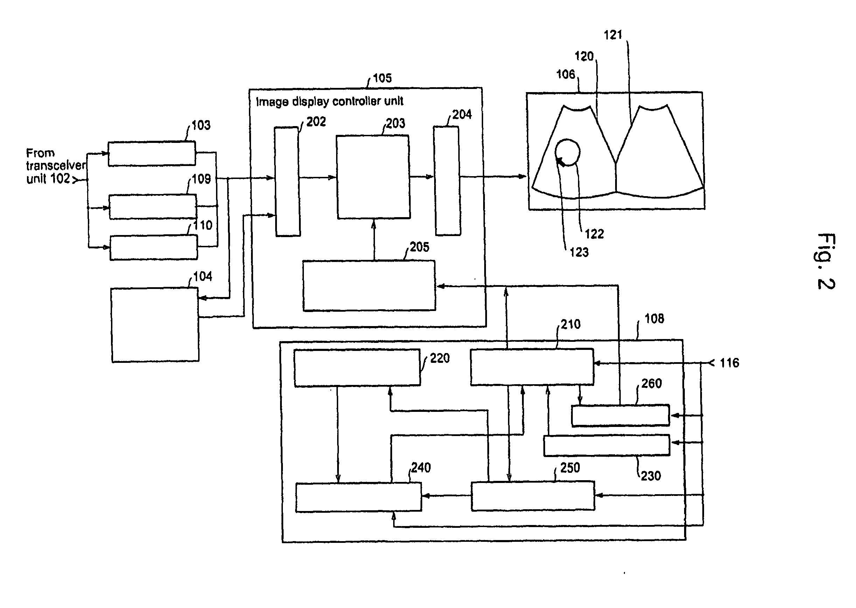

Fig. 1 shows a schematic block diagram indicative of the overview of ultrasonic imaging apparatus in accordance with a preferred embodiment of the present invention. The ultrasonic imaging apparatus has a probe 101, a transceiver unit 102, a B mode processing unit 103, a Doppler processing unit 109, a color flow mapping (CFM) processing unit 110, an image memory unit 104, an image display controller unit 105, a display unit 106, a selector means 116, and a controller unit 108. It is noted here that in Fig. 1 and Fig. 2 which will be described hereinbelow, the signal lines for carrying image information or selection information are indicated by solid lines, while the signal lines for carrying the control information is indicated by dotted lines.

The probe unit 101 is a unit for sending and receiving ultrasonic waves, namely repeatedly transmits the ultrasonic waves to a specific direction in the imaging section of a living body, and receives the ultrasonic signal reflected from the body as the sonic line of time sequence. On the other hand the probe unit 101 sequentially switches the transmission direction of ultrasonic waves to perform the electronic scan. Although not shown in the figure, the probe unit 101 has piezoelectric modules arranged as an array. The probe unit 101 in addition is not limited to be an electronic scanning probe, but may be a probe for mechanical scan.

The transceiver unit 102 is connected to the probe unit 101 with a coaxial cable and generates electric signals for driving the piezoelectric modules in the probe unit 101. The transceiver unit 102 also performs first stage amplification of received ultrasonic signals.

The B mode processing unit 103 generates in real time basis a B mode image from the ultrasonic signals amplified by the transceiver unit 102. More specific processing details may be such as the delayed addition of received ultrasonic signals, Analog-to-Digital conversion, writing digitized information after conversion as the B mode image information to the image memory unit 104 to be described below and so on.

The Doppler processing unit 109 extracts the phase modulation information at the timings corresponding to the sampled area set in the B mode image from within the ultrasonic signals amplified by the transceiver unit 102, to generate as a Doppler image the information on the flow associated with various points in the imaged section in real time basis, such as the velocity, power value, and variance.

The CFM processing unit 110 generates a CFM image, which illustrates the blood flow information included in the ultrasonic signal as red for incoming flow to the probe unit 101 and blue for outgoing flow from the probe unit 101. Note that the term ultrasonic image herein below refers to the image information obtained by transmitting and receiving ultrasonic waves to a subject, including the B mode image described above, CFM image, as well as Doppler image.

The image memory unit 104 is an image memory for accumulating ultrasonic image information such as including B mode image information generated in the B mode processing unit 103, CFM image information generated in the CFM processing unit 110, as well as Doppler image information generated in the Doppler processing unit 109.

The image display controller unit 105 performs control of conversion of displaying frame rate of the ultrasonic image information, mode of image display, and image layout in the display unit 106.

The display unit 106 uses a CRT (cathode ray tube) or LCD (liquid crystal display) and the like to provide visual display of a plurality of image information controlled by the image display controller unit 105 for the operator.

The selector means 116 includes an input unit 107 and a track-ball 111 for inputting control information to the controller unit 108. The input unit 107 may include a keyboard, to communicate the operational input signals from the operator to the controller unit 108. The track-ball 111 is a pointing device for controlling the cursor position on the display screen of the display unit 106. The measurement pattern as will be described below may be specified by the cursor to be displayed in the ultrasonic image on the display unit 106.

The controller unit 108 controls the operation of various units of the ultrasonic imaging apparatus above based on the operational input signal supplied from the input unit 107, and preloaded program and data, to display the ultrasonic image such as B mode image to the display unit 106.

Fig. 2 shows a functional block diagram indicative of a display function section of ultrasonic image and measurement pattern in the controller unit 108 and the image display controller unit 105.

The image display controller unit 105 includes a display image generator unit 202, a frame memory 203, a display image loader unit 204, and a measurement pattern display unit 205. The frame memory 203 is a memory for accumulating display screen information to be displayed on the display unit 106. The display image generator unit 202 generates the image mode to be displayed on the display unit 106 based on the ultrasonic image information from the B mode processing unit 103 and the like. The display image generator unit 202 then writes the image information into the address corresponding to the display position in the frame memory 203. The display image loader unit 204 reads the image information written into the frame memory 203 to display on the display unit 106.

The measurement pattern display unit 205 writes the measurement pattern into the display position in the frame memory 203 based on the measurement pattern information about the location and form of the measurement pattern derived from the controller unit 108. The measurement pattern then will be displayed on the display unit 106. In the display unit 106 shown in Fig. 2, there are two B mode images 120 and 121 shown as examples of ultrasonic images, in addition a cursor 123 and a circle 122 as an example of measurement pattern are shown in the B mode image 120.

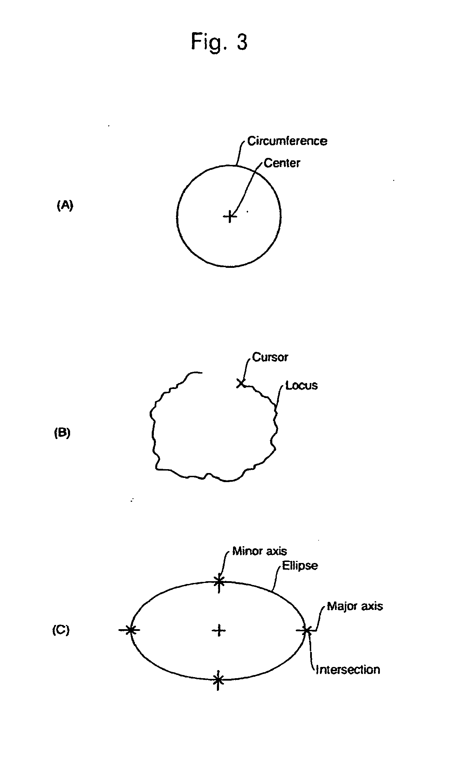

An example of measurement pattern to be shown on the display unit 106 is shown in Fig. 3. Fig. 3 (A) is an example of circle, which is defined by the center position of circle and the position of circumference, both specified by the cursor 123 in the ultrasonic image. Fig. 3 (B) is an example of trace, which displays the trace of the cursor 123 in the ultrasonic image, moved by the track-ball 111 or the like. Fig. 3 (C) is an example of ellipse, the position and the size of which are specified by the cursor 123 in the ultrasonic image.

In addition, a caliper indicative of a line segment may be used for a measurement pattern.

The controller unit 108 includes a measurement pattern information buffer memory 210, a measurement pattern form information storage unit 220, a measurement pattern moving means 230, a measurement pattern reproduction means 240, a measurement pattern storage means 250, and an image measurement means 260. The measurement pattern information buffer memory 210 is specified by the cursor 123 at the time when the measurement pattern set mode is selected from the input unit 107. It is a buffer memory for storing the measurement pattern information on the position and form of the measurement pattern. The contents of this buffer memory is update whenever measurement pattern information is input from the selector means 116 and is transferred to the measurement pattern display unit 205.

In the measurement pattern information buffer memory 210, the information of two positional data items of the center position information and the circumference position information of a circle is stored when the circle shown in Fig. 3 (A) is selected; the information of positional data items of many points on the trace is stored when the trace is selected as shown in Fig. 3 (B); and the information of four intersectional positions where the major and minor axes of an ellipse intersect is stored when the ellipse is selected as shown in Fig. 3 (C).

The measurement pattern storage means 250 selects a measurement pattern present at the position of the cursor 123 in the ultrasonic image from the measurement pattern information buffer memory 210 when the measurement pattern edit mode is selected from the input unit 107, and extracts only the morphological information from the measurement pattern information, to store the information in the measurement pattern form information storage unit 220 which is the memory. For instance, when the measurement pattern is a circle, the circle as well as the radius or the diameter of the circle are stored as the form information; information on relative position of the positional information of many points on the trace is stored as the form information when the measurement pattern is a trace; and the information on relative position of the positional information of intersections is stored when the measurement pattern is an ellipse.

The measurement pattern reproduction means 240 is responsive to the selection signal from the input unit 107 to read the form information of the measurement pattern stored in the measurement pattern form information storage unit 220 when the measurement pattern edit mode is selected from the input unit 107, to add the positional information corresponding to the cursor position in the B mode image 120 or 121, and to write it into the measurement pattern information buffer memory 210. Then the measurement pattern display unit 205 generates the measurement pattern in the frame memory 203 to display at the position of the cursor 123 in the display unit 106.

The measurement pattern moving means 230 changes the measurement pattern positional information in the measurement pattern information buffer memory 210, when the measurement pattern edit mode is selected from the input unit 107, in response to the movement of the cursor 123 position, for example with respect to the measurement pattern at the cursor 123 position in the B mode image 120 or 121, to move the measurement pattern so as to follow the position where the cursor 123 is present. The measurement pattern moving means 230 also performs the operation such as rotation, invert of the measurement pattern under the direction from the selector means 116.

The image measurement means 260 performs various measurements by using the position and form information in the measurement pattern information buffer memory 210. For instance, when the circle is selected, it calculates the area of the section encompassed by the circle; when the trace is selected it calculates the area of the section encompassed by the trace; when the ellipse is selected, it calculates the area of the section encompassed by the ellipse. The image measurement means 260 is connected to the measurement pattern display unit 206 of the image display controller unit 105 to transfer the calculation result described above to the measurement pattern display unit 206 to display on the display unit 106.

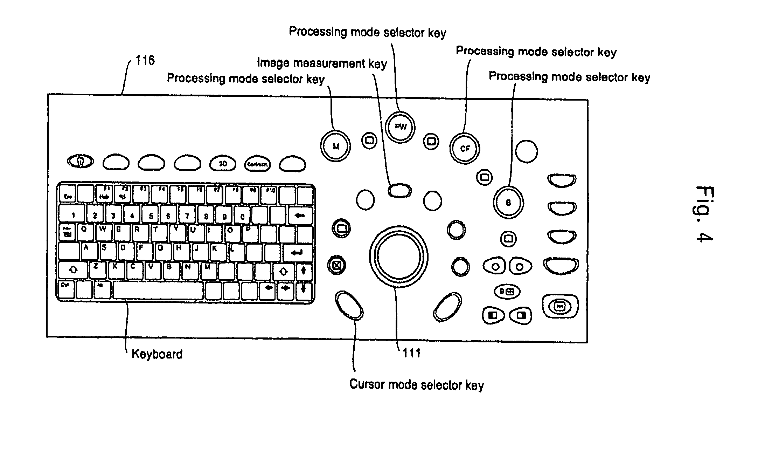

Fig. 4 shows an example of the track-ball 111 and the input unit 107 of the selector means 116. On the left side of the selector means 116 a keyboard is provided and on the right side a track-ball 111 is provided for controlling the position of the cursor 123. Around the track-ball 111 processing mode selection key such as B mode processing and M mode processing in relation to the Doppler processing are placed, and among those keys there are a cursor mode selection key for switching the modes between the measurement pattern set mode and measurement pattern edit mode of the cursor 123, and an image measurement key for invoking the image measurement means 260.

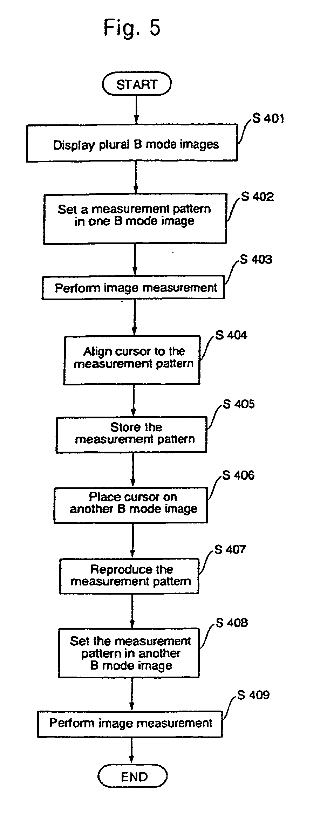

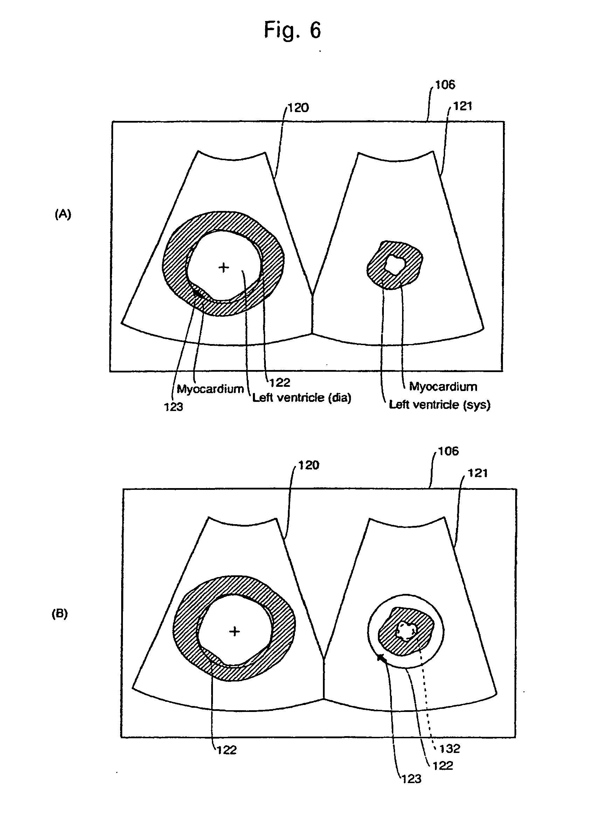

Next, the detailed operation of the measurement pattern storage means 250 and the measurement pattern reproduction means 240 in the controller unit 108 will be described in greater details with reference to Fig. 5. Fig. 5 shows a flow chart illustrating the operation of the measurement pattern storage means 250 and the measurement pattern reproduction means 240 of the controller unit 108. First, the operator instructs to display on the display unit 106 a plurality of still ultrasonic images, for example two B mode images of an imaging site which changes in morphology along with time course (step S401). Fig. 6 shows an example of displaying two B mode images. The B mode images 120 and 121 shown in Fig. 6 illustrates schematically the brachyaxial tomographic plane of the left ventricle of a heart, captured by the ultrasonic imaging apparatus, in which the B mode image 120 of the left hand side shows the left ventricular diastolic phase while the B mode image 121 of the right hand side shows the left ventricular systolic phase. It should be noted here that these two images are selected among many B mode images of cardiac region of a subject that have been imaged in advance using the ultrasonic imaging apparatus and stored in the image memory unit 104.

Then, the operator uses the selector means 116 to select the measurement pattern set mode to set a measurement pattern in the B mode image 120 (step S402). Fig. 6 (A) shows an example of the measurement pattern to be set. Here a circle 122 as measurement pattern is illustrated, which is aligned to the inner wall position of the left ventricle in diastolic phase. The position of the circle 122 may be fitted to the left ventricle by using the measurement pattern moving means 230.

The operator then uses the image measurement means 260 to measure on the image the surface area of inner wall of the left ventricle in diastolic phase based on the circle 122 superimposed thereon (step S403) to obtain some numerical data such as surface area. Although the circle 122 is used as the measurement pattern for the sake of clarity, the trace may be much preferable for the candidate of measurement pattern in order to perform an image measurement of fine precision.

The operator then uses the selector means 116 to select the measurement pattern edit mode to move the cursor 123 on the circle 122 to match with the measurement pattern (step S404), to invoke the measurement pattern storage means 250 by a predetermined keyboard operation such as for example by pressing the CTRL and C keys on the keyboard at the same time, to copy or store the morphological information of the circle 122 to the measurement pattern form information storage unit 220 (step S405).

The operator then moves the cursor 123 to another image, namely the B mode image 121 and place the cursor 123 on the position where the circle 122 is to be reproduced, namely pasted (step S406). The operator thereafter invokes the measurement pattern reproduction means 240 by a predetermined keyboard operation such as for example by pressing the CTRL and V key on the keyboard at the same time, to read out the morphological information of the circle 122 from the measurement pattern form information storage unit 220. Then the measurement pattern reproduction means 240 writes the measurement pattern on the position and form of the circle 122 into the measurement pattern information buffer memory 210 based on the positional information on the cursor 123 from the selector means 116 to paste, namely, reproduce the circle 122 at the position of the cursor 123 in the B mode image 121 (step S407). Fig. 6 (B) shows the circle 122, which is the measurement pattern being reproduced in the B mode image 121. The circle 122 is placed so as to encompass the left ventricle in diastolic phase. The circle 122 may be placed at the optimal location by using the measurement pattern moving means 230 in order to compare with the left ventricle in diastolic phase. A plurality of circles 122 may be reproduced in a B mode image 121 by the similar operation.

The operator thereafter selects the measurement pattern set mode by means of the selector means 116 to set a measurement pattern for example a circle 132 on the left ventricle in the B mode image 121, which indicates the left ventricular systolic phase, while referencing to the circle 122 which is the reproduced measurement pattern (step S408). Fig. 6 (B) shows the circle 132, additionally placed measurement pattern in the B mode image 121, by dotted line. The circle 132 is placed so as to superpose on the inner wall of the left ventricle in systolic phase.

The operator then uses the image measurement means 260 to perform the image measurement such as the area of inner wall of the left ventricle in systolic phase with the circle 132 (step S409) to obtain the numerical data of the left ventricle in systolic phase. The left ventricle in diastolic phase is shown in the B mode image 121 of the systolic phase with the pasted circle 122, so as to allow facilitating the comparison of morphology of the left ventricle in systolic phase to the left ventricle in diastolic phase in an easier and visually reliable manner.

As have been described above, in accordance with the preferred embodiment, since the measurement pattern storage means 250 stores the circle 122 indicative of the size of the inner wall of left ventricle, specified in the B mode image 120 which shows the left ventricular diastolic phase, and the measurement pattern reproduction means 240 reproduces the circle 122 in the B mode image 121 indicative of the left ventricular systolic phase, the comparison of morphology of the inner wall of left ventricle in diastolic and systolic phase can be performed when specifying the circle 132 indicative of the size of the inner wall of left ventricle in systolic phase in the B mode image 121, in an easer and visually reliable manner along with the numerical data from the image measurement means 260 to provide an appropriate determination of the numerical data.

In accordance with the preferred embodiment, although there is only one measurement pattern set in the B mode images 120 and 121, when a plurality of measurement patterns is set in the images, these measurement patterns are specified and stored in the order of sequential number which is served as identification information, to sequentially reproduce in the order of sequential number, so as to perform the storage and reproduction of measurement patterns in an efficient manner.

The measurement pattern storage means 250 in such a case may have a sequential storage means for labeling a sequential number to a plurality of measurement patterns to be stored, while the measurement pattern reproduction means 240 may have a sequential reproduction means for sequentially reading and reproducing thus numbered measurement pattern morphological information.

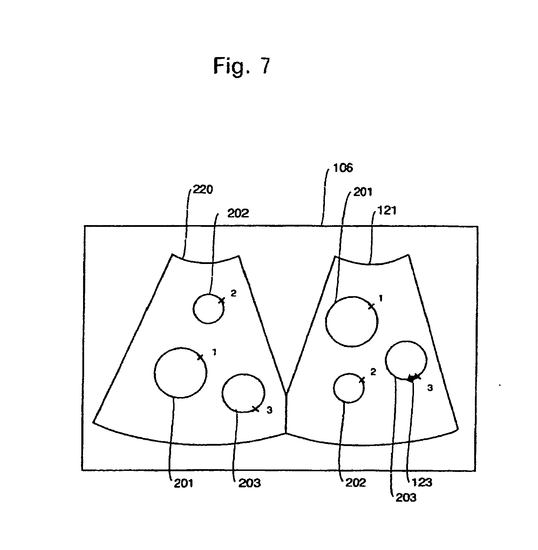

Fig. 7 shows an example of the measurement patterns 211 to 213 being set on the B mode image 220 displayed on the display unit 106. The measurement patterns 211 to 213 are displayed with added sequence number "1" to "3" which are identification information. At the time when selecting one by one the position of the measurement patterns 211 to 213, by means of the cursor 123, the sequential storage means in the measurement pattern storage means 250 stores into the measurement pattern form information storage unit 220 the morphological information of the measurement patterns 211 to 213 along with the number information "1" to "3". At this point the number information is transferred from the measurement pattern storage means 250 to the measurement pattern reproduction means 240.

The sequential reproduction means in the measurement pattern reproduction means 240 will reproduce the measurement patterns 211 to 213 in the order of number information "1" to "3" for example at the location specified in the B mode image 221 by using the cursor 123. In the B mode image 221 of Fig. 7 thus reproduced measurement patterns 211 to 213 are shown.

In accordance with the preferred embodiment, although there is only one measurement pattern set in the B mode images 120 and 121, there may be cases where a plurality of measurement patterns are set in these images, in which cases the measurement patterns are stored at once and reproduced at once, so as to perform the storage and reproduction in a more effective manner.

The measurement pattern storage means 250 in such a case may have a batch storage means for storing a plurality of measurement patterns at once, and the measurement pattern reproduction means 240 may have a batch reproduction means for reproducing at once a plurality of measurement patterns that have been stored in a batch.

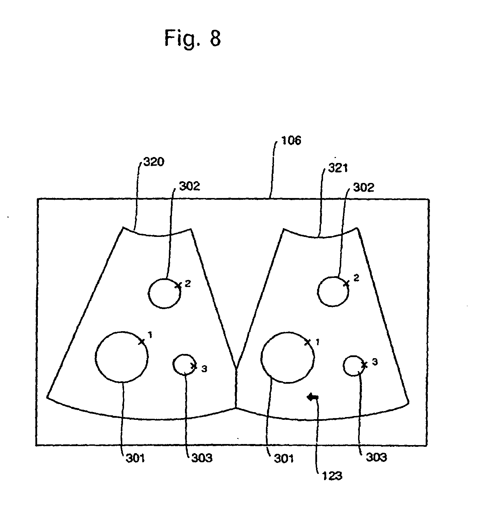

Fig. 8 shows an example of circles 301 to 303 being set as measurement patterns in a B mode image 320 displayed on the display unit 106. In this example by specifying a B mode image 320 by means for example of the cursor 123, the batch storage means of the measurement pattern storage means 250 will store in the measurement pattern form information storage unit 220 the morphological information of the circles 301 to 303 along with the positional information.

Furthermore, the batch reproduction means of the measurement pattern reproduction means 240 may reproduce the circles 301 to 303 at once on a B mode image 321 by specifying the B mode image 321 by means for example of the cursor 123. In the B mode image 321 of Fig. 8 the circles 301 to 303, which have been reproduced at once, are shown.

In addition in accordance with the preferred embodiment, it has been described that the circle 122 set in the B mode image 120 of diastolic phase and the circle 122 reproduced in the B mode image 121 of systolic phase are the same, however the circle 122 reproduced in the B mode image 121 of systolic phase may also have a different color or line width to display as identifiable. By doing this the circle 132 and circle 122 both set in the B mode image 121 of systolic phase can be clearly distinguished each from the other to allow the morphological comparison to be effectuate much easier.

Also in accordance with the preferred embodiment, although the B mode images 120 and 121 has the same display magnification rate of the visualized B mode images, the size of the measurement patterns to be reproduced can be altered in response to the display magnification rate, when the display magnification rate of images are different, to match with the size of B mode images. In this example the measurement pattern reproduction means 240 has a magnification rate changing means, for altering the size of measurement patterns read from the measurement pattern form information storage unit 220 in correspondence with the display magnification rate of the B mode image, such that for example if the B mode image to which a measurement pattern is to be reproduced is displayed magnified to twofold, the measurement pattern to be reproduced in this B mode image will be accordingly magnified to twofold.

Furthermore in accordance with the preferred embodiment although there have been shown examples of left ventricular images in diastolic and systolic phases for the B mode images 120 and 121 that changes in morphology along with time course, images of tumor prior to and after the surgical intervention thereof may be similarly used.

## Claims

1. An ultrasonic imaging apparatus which displays ultrasonic image of a subject, and also displays a measurement pattern, in said displayed ultrasonic image based on measurement pattern information with respect to a location and a form of the measurement pattern to be input, to perform the image measurement of said ultrasonic image, said ultrasonic imaging apparatus comprising:
a selector device (116) for specifying said location in a first ultrasonic image;
image measurement means (260) for calculating morphological information of said measurement pattern;
a measurement pattern storage device (250) for storing said morphological information of said measurement pattern when a measurement pattern is present at the specified location;
a measurement pattern reproduction device (240) for reproducing the stored measurement pattern of the morphological information at a location in said first ultrasonic image specified using said selector device (116);
a measurement pattern information buffer memory (210) for storing measurement pattern information on the position and form of the measurement pattern;
wherein said measurement pattern comprises a circle, an ellipse, or a trace of the displayed image of the subject;
and said morphological information of said measurement pattern is the area of the section encompassed by said circle, ellipse or trace;
further comprising measurement pattern moving means (230) for changing the measurement pattern positional information in the measurement pattern information buffer memory (210) and for rotating or inverting the measurement pattern in response to input from the selector device (116); and wherein
said measurement pattern reproduction device (240) is for reproducing the stored measurement pattern of the morphological information from the first image at a location in the first ultrasonic image newly specified using said selector device (116), or in a second ultrasonic image different from said first ultrasonic image.

2. An ultrasonic imaging apparatus according to claim 1, wherein:
said measurement pattern storage device (250) performs said storage in association with said selection.

3. An ultrasonic imaging apparatus according to claim 1 or 2, wherein:
said measurement pattern reproduction device (240) performs said reproduction for a plurality of times.

4. An ultrasonic imaging apparatus according to any one of claims 1 to 3,
wherein:
said measurement pattern storage device (250) includes a sequential storage device for sequentially storing a plurality of said measurement patterns with identification information when there is a plurality of measurement patterns present in said first ultrasonic image.

5. An ultrasonic imaging apparatus according to claim 4, wherein:
said identification information is number information indicative of the sequential number of storing.

6. An ultrasonic imaging apparatus according to claim 4 or 5, wherein:
said measurement pattern reproduction device (240) includes a sequential reproduction device for sequentially reproducing said measurement patterns based on said identification information.

7. An ultrasonic imaging apparatus according to any one of claims 1 to 3,
wherein:
said measurement pattern storage device (250) includes a batch storage device for storing at once all of measurement patterns present in said first ultrasonic image.

8. An ultrasonic imaging apparatus according to claim 7, wherein:
said measurement pattern reproduction device (240) includes a batch reproduction device for reproducing at once said plurality of measurement patterns.

9. An ultrasonic imaging apparatus according to any one of claims 1 to 8,
wherein:
said measurement pattern reproduction device (240) includes a magnification rate changer device for changing, when a display magnification rate of the ultrasonic image in which the measurement pattern is to be reproduced is different from the display rate of the ultrasonic image used for storing, a size of said measurement pattern to be reproduced in association with the change of said display magnification rate.

10. An ultrasonic imaging apparatus according to any one of claims 1 to 9,
wherein:
said measurement pattern reproduction device (240) performs identifiable indication for distinguishing said reproduced measurement pattern from the measurement pattern based on said measurement pattern information to be input.

## Patentansprüche

1. Ultraschall-Bildgebungsvorrichtung, die ein Ultraschall-Bild von einem Objekt darstellt und die ebenfalls ein Ausmessungsmuster in dem dargestellten Ultraschall-Bild darstellt, das auf einer Ausmessungsmuster-Information bezogen auf einen Ort und eine Form des einzugebenden Ausmessungsmusters basiert, um die Bild-Ausmessung des Ultraschall-Bildes durchzuführen, wobei die Ultraschall-Bildgebungsvorrichtung aufweist:
eine Auswahl-Einrichtung (116) zum Spezifizieren des Ortes in einem ersten Ultraschall-Bild;
Bild-Ausmessungsmittel (260) zum Berechnen von morphologischer Information aus dem Ausmessungsmuster;
eine Ausmessungsmuster-Speichereinrichtung (250) zum Speichern der morphologischen Information aus dem Ausmessungsmusters, wenn ein Ausmessungsmuster an dem spezifizierten Ort vorliegt;
eine Ausmessungsmuster-Wiedergabeeinrichtung (240) zum Wiedergeben des gespeicherten Ausmessungsmusters der morphologischen Information über einen Ort in dem ersten Ultraschall-Bild unter Verwendung der Einrichtung (116);
ein Ausmessungsmuster-Informations-Zwischenspeicher (210) zum Speichern der Ausmessungsmuster-Information über die der Position und der Form des Ausmessungsmusters;
worin das Ausmessungsmuster einen Kreis, eine Ellipse oder einen Umriss des dargestellten Bildes des Objektes aufweist; und
die morphologische Information des Ausmessungsmusters gleich der Fläche des Bereiches ist, der von dem Kreis, der Ellipse oder dem Umriss umfasst wird;
ferner aufweisend Ausmessungsmuster-Bewegungsmittel (230) zum Ändern der Positionsinformation in dem Ausmessungsmuster-Informations-Zwischenspeicher (210) und zum Drehen oder Invertieren des Ausmessungsmusters als Antwort auf eine Eingabe der Auswahl-Einrichtung (116); und worin
die Ausmessungsmuster-Wiedergabeeinrichtung (240) zur Wiedergabe des gespeicherten Ausmessungsmusters der morphologischen Information von dem ersten Bild an einem Ort in dem ersten Ultraschall-Bild dient, das neu unter Verwendung der Auswahl-Einrichtung (116) spezifiziert wurde, oder in einem zweiten Ultraschall-Bild, das verschieden von dem ersten Ultraschall-Bild ist.

2. Ultraschall-Bildgebungsvorrichtung gemäß Anspruch 1,
worin:
die Ausmessungsmuster-Speichereinrichtung (250) die Speicherung in Verbindung mit der Auswahl durchführt.

3. Ultraschall-Bildgebungsvorrichtung gemäß Anspruch 1 oder 2, worin:
die Ausmessungsmuster-Wiedergabeeinrichtung (240) die Wiedergabe zu mehreren Zeitpunkten durchführt.

4. Ultraschall-Bildgebungsvorrichtung gemäß Anspruch 1 bis 3, worin:
die Ausmessungsmuster-Speichereinrichtung (250) eine sequentielle Speichereinrichtung zum sequentiellen Speichern von mehreren der Ausmessungsmuster mit Identifikationsinformationen enthält, wenn mehrere Ausmessungsmuster in dem ersten Ultraschall-Bild vorliegen.

5. Ultraschall-Bildgebungsvorrichtung gemäß Anspruch 4,
worin:
die Identifikationsinformation eine Information der Anzahl ist, die anzeigend für die sequentielle Anzahl der Speicherung ist.

6. Ultraschall-Bildgebungsvorrichtung gemäß Anspruch 4 oder 5, worin:
die Ausmessungsmuster-Wiedergabeeinrichtung (240) eine sequentielle Wiedergabeeinrichtung zum sequentiellen Wiedergeben des Ausmessungsmusters auf der Basis der Identifikationsinformation ist.

7. Ultraschall-Bildgebungsvorrichtung gemäß Anspruch 1 bis 3, worin:
die Ausmessungsmuster-Speichereinrichtung (250) eine Batch-Speichereinrichtung zum gleichzeitigen Speichern von allen Ausmessungsmustern enthält, die in dem ersten Ultraschall-Bild vorliegen.

8. Ultraschall-Bildgebungsvorrichtung gemäß Anspruch 7,
worin:
die Ausmessungsmuster-Wiedergabeeinrichtung (240) eine Batch-Wiedergabeeinrichtung zum gleichzeitigen Wiedergeben der mehreren Ausmessungsmuster enthält.

9. Ultraschall-Bildgebungsvorrichtung gemäß Anspruch 1 bis 8, worin:
die Ausmessungsmuster-Wiedergabeeinrichtung (240) eine Verstärkungsraten-Veränderungseinrichtung zum Ändern aufweist, wenn eine Darstellungsvergrößerungsrate des wiederzugebenden Ultraschall-Bildes, in dem das Ausmessungsmuster liegt, verschieden von der Darstellungsrate des Ultraschall-Bildes ist, das für die Speicherung einer Größe des wiederzugebenden Ausmessungsmusters in Verbindung mit der Änderung der Darstellungsvergrößerungsrate verwendet wird.

10. Ultraschall-Bildgebungsvorrichtung gemäß Anspruch 1 bis 9, worin:
die Ausmessungsmuster-Wiedergabeeinrichtung (240) die identifizierbare Anzeige zum Unterscheiden des wiedergegebenen Ausmessungsmusters von dem Ausmessungsmuster auf der Basis der Ausmessungsmuster-Information durchführt, die eingegeben wird.

## Revendications

1. Appareil d'imagerie ultrasonore affichant une image ultrasonore d'un sujet et affichant également un motif de mesure, dans ladite image ultrasonore affichée, basé sur des informations de motif de mesure relatives à une position et une forme du motif de mesure devant être entrées, afin d'effectuer la mesure d'image de ladite image ultrasonore, ledit appareil d'imagerie ultrasonore comprenant :
un dispositif sélecteur (116) pour spécifier ladite position dans une première image ultrasonore;
un moyen de mesure d'image (260) pour calculer une information morphologique dudit motif de mesure;
un dispositif de stockage de motif de mesure (250) pour stocker ladite information morphologique dudit motif de mesure lorsqu'un motif de mesure est présent à la position spécifiée;
un dispositif de reproduction de motif de mesure (240) pour reproduire le motif de mesure stocké de l'information morphologique à une position dans ladite première image ultrasonore spécifiée à l'aide dudit dispositif sélecteur (116);
une mémoire tampon d'informations de motif de mesure (210) pour stocker des informations de motif de mesure sur la position et la forme du motif de mesure;
dans lequel ledit motif de mesure comprend un cercle, une ellipse ou un tracé de l'image affichée du sujet;
et ladite information morphologique dudit motif de mesure est l'aire de la section entourée par ledit cercle, ellipse ou tracé;
comprenant en outre un moyen de déplacement de motif de mesure (230) pour changer les informations de position de motif de mesure dans la mémoire tampon d'informations de motif de mesure (210) et pour faire tourner ou inverser le motif de mesure en réponse à une entrée provenant du dispositif sélecteur (116); et dans lequel
ledit dispositif de reproduction de motif de mesure (240) sert à reproduire le motif de mesure stocké de l'information morphologique provenant de la première image à une position dans la première image ultrasonore nouvellement spécifiée à l'aide dudit dispositif sélecteur (116), ou dans une seconde image ultrasonore différente de ladite première image ultrasonore.

2. Appareil d'imagerie ultrasonore selon la revendication 1,
dans lequel:
ledit dispositif de stockage de motif de mesure (250) effectue ledit stockage en association avec ladite sélection.

3. Appareil d'imagerie ultrasonore selon la revendication 1 ou 2, dans lequel:
ledit dispositif de reproduction de motif de mesure (240) effectue ladite reproduction une pluralité de fois.

4. Appareil d'imagerie ultrasonore selon l'une quelconque des revendications 1 à 3, dans lequel:
ledit dispositif de stockage de motif de mesure (250) comprend un dispositif de stockage séquentiel pour stocker séquentiellement une pluralité desdits motifs de mesure avec une information d'identification lorsqu'il existe une pluralité de motifs de mesure présents dans ladite première image ultrasonore.

5. Appareil d'imagerie ultrasonore selon la revendication 4,
dans lequel:
ladite information d'identification est une informations de numéro indicative du numéro d'ordre de stockage.

6. Appareil d'imagerie ultrasonore selon la revendication 4 ou 5, dans lequel:
ledit dispositif de reproduction de motif de mesure (240) comprend un dispositif de reproduction séquentielle pour reproduire séquentiellement lesdits motifs de mesure sur la base de ladite information d'identification.

7. Appareil d'imagerie ultrasonore selon l'une quelconque des revendications 1 à 3, dans lequel:
ledit dispositif de stockage de motif de mesure (250) comprend un dispositif de stockage de lot pour stocker en une fois la totalité des motifs de mesure présents dans ladite première image ultrasonore.

8. Appareil d'imagerie ultrasonore selon la revendication 7,
dans lequel:
ledit dispositif de reproduction de motif de mesure (240) comprend un dispositif de reproduction de lot pour reproduire en une fois ladite pluralité de motifs de mesure.

9. Appareil d'imagerie ultrasonore selon l'une quelconque des revendications 1 à 8, dans lequel:
ledit dispositif de reproduction de motif de mesure (240) comprend un dispositif changeur de coefficient d'agrandissement pour changer, lorsqu'un coefficient d'agrandissement d'affichage de ladite image ultrasonore dans laquelle le motif de mesure doit être reproduit est différent du coefficient d'agrandissement d'affichage de l'image ultrasonore utilisée pour le stockage, une dimension dudit motif de mesure devant être reproduit en association avec le changement dudit coefficient d'agrandissement d'affichage.

10. Appareil d'imagerie ultrasonore selon l'une quelconque des revendication 1 à 9, dans lequel:
ledit dispositif de reproduction de motif de mesure (240) effectue une indication identifiable pour distinguer ledit motif de mesure reproduit par rapport au motif de mesure basé sur desdites informations de motif de mesure devant être entrées.
